# EUROPEAN PATENT SPECIFICATION

(11) **EP 2 350 646 B1**
(45) Date of publication and mention of the grant of the patent: **13.01.2016**
(21) Application number: 09760590.1
(22) Date of filing: 27.10.2009
(51) Int. Cl.: G01N 33/50, G01N 33/68

(54) **METHODS AND KITS FOR THE RAPID DETERMINATION OF PATIENTS AT HIGH RISK OF DEATH DURING SEVERE SEPSIS AND SEPTIC SHOCK**
VERFAHREN UND KITS ZUR SCHELLEN BESTIMMUNG VON PATIENTEN MIT HOHEM STERBERISIKO BEI SCHWERER SEPSIS UND SEPTISCHEM SCHOCK
MÉTHODES ET TROUSSES PERMETTANT D'IDENTIFIER RAPIDEMENT LES PATIENTS À HAUT RISQUE DE DÉCÈS EN CAS DE SEPSIE SÉVÈRE ET DE CHOC SEPTIQUE

(30) Priority: 28.10.2008 US 259596; 09.03.2009 US 158520 P
(43) Date of publication of application: 03.08.2011
(73) Proprietor: Assistance Publique - Hôpitaux de Paris, 75004 Paris (FR)
(72) Inventor: PAYEN DE LA GARANDERIE, Didier, 75018 Paris (FR); LUKASZEWICZ, Anne-Claire, 60500 Chantilly (FR)
(74) Representative: Marcadé, Véronique
(86) International application number: PCT/IB2009/007493
(87) International publication number: WO 2010/049818

(56) References cited:
- EP-A1- 1 950 310
- EP-A1- 2 085 486
- WO-A1-2008/104321
- US-A1- 2006 035 221
- US-A1- 2006 234 295
- DIDIER PAYEN ET AL: "Gene profiling in human blood leucocytes during recovery from septic shock" INTENSIVE CARE MEDICINE, SPRINGER, BERLIN, DE, vol. 34, no. 8, 5 April 2008 (2008-04-05), pages 1371-1376, XP019619034 ISSN: 1432-1238 cited in the application
- IKEMOTO MASAKI ET AL: "New ELISA system for myeloid-related protein complex (MRP8/14) and its clinical significance as a sensitive marker for inflammatory responses associated with transplant rejection" CLINICAL CHEMISTRY, AMERICAN ASSOCIATION FOR CLINICAL CHEMISTRY, WASHINGTON, DC, vol. 49, no. 4, 1 April 2003 (2003-04-01), pages 594-600, XP002456282 ISSN: 0009-9147 cited in the application
- VOGL T ET AL: "Mrp8 and Mrp14 are endogenous activators of Toll-like receptor 4, promoting lethal, endotoxin-induced shock" NATURE MEDICINE 200709 GB, vol. 13, no. 9, September 2007 (2007-09), pages 1042-1049, XP002568723 cited in the application

## Description

### FIELD OF THE INVENTION

The present invention relates to the field of treatment of serious medical syndromes such as severe sepsis or septic shock. In particular, the present invention provides methods and kits to obtain an early evaluation of mortality risk and help therapeutic decisions for patients in severe sepsis with two organ failures, for example for patients in septic shock.

### BACKGROUND AND PRIOR ART

Septic shock is the most severe clinical presentation of sepsis, with a poor prognosis despite intensive therapeutic support and anti-infectious strategy to eradicate the infection foci. The sepsis syndrome is defined as symptoms related to the host response to abnormal presence of micro-organisms (bacteria, viruses or parasites) or their antigenic fractions. The local infection might spread out for different reasons to the whole body, with a particular activation of blood immune cells controlling the innate immunity during the early phase and activation of the adaptive immunity in a second time. Such an intense immune activation in blood may in turn target organs that were not initially concerned by the initial infection, leading to immune toxicity and dysfunction of these organs. The high mortality rate of septic shock (around 50%) results from a combination of organ failures, comorbidities and virulence of micro-organisms. Death may occur at different times of evolution, most often during the first week despite intensive resuscitation.

A number of studies examining therapeutic interventions have generated important but controversial results, such as the use of corticosteroids (Annane et al., 2002; Sprung et al., 2008), activated protein C (Bernard et al., 2001), and tight glycaemic control (van den Berghe et al., 2001). The absence of an early, specific and sensitive marker for determining prognosis thus creates difficulties not only for clinicians with regard to prescription of expensive drugs and futile maintenance of life support, but also for trial investigators with regard to the selection of patients for study inclusion. Some promising markers have been tested, such as procalcitonin (Clec'h et al., 2004), HLA-DR (Monneret et al., 2006), IL-6 (Abraham et al., 2001), and soluble triggering receptor expressed on myeloid cells-1 (sTREM) (Gibot et al., 2005); but results are conflicting.

Recently, a novel group of molecules - known variously as "endokines", "alarmins" or "damage-associated molecular pattern proteins (DAMPs)" - that are released by activated or damaged cells under conditions of cell stress has been investigated (Oppenheim et al., 2007). Among these, phagocytic S100 proteins, members of the calgranulin family that mediate inflammatory responses (Vogl et al., 2007) and organ function (Boyd et al., 2008) appear to be of importance in sepsis. An emerging concept of pattern recognition involves the multi-ligand receptor for advanced glycation end products (RAGE) and Toll-like receptors (TLRs) in sensing not only pathogen-associated molecular patterns (PAMPs) but also endogenous DAMPs, including the S100 proteins (Brunn and Platt, 2006; Foell et al., 2007).

Overexpression of the genes of S100A8 and A9 has been shown in mice submitted to lipopolysaccharide (LPS) or caecal ligation and puncture (CLP) challenge (Vogl et al., 2007), in cardiomyocytes incubated with LPS (Boyd et al., 2008), in kidney tissue after ischaemia/reperfusion (Grigoryev et al., 2008), in healthy volunteer leucocytes after LPS injection (Talwar et al., 2006) and in septic shock patients (Payen et al., 2008). Since S100A8 and S100A9 proteins are found in both intracellular and extracellular spaces, they could potentially act both within the cell and in an autocrine or paracrine manner. In mice with septic shock, there were high levels of both gene expression and the proteins (Vogl et al., 2007). Few studies have examined cellular or extracellular levels of these proteins in human septic shock.

WO 2008/104321 describes the use of the peptide procalcitonin, as a biomarker for early diagnosis, monitoring of treatment and monitoring of the progression of systemic and local infections, in particular bacterial infections and sepsis. S100A proteins are mentioned in a group of potential additional markers which can be used in combination with procalcitonin. EP 1 950 310, US 2006/0234295 and US 2006/0035221 also list S100A proteins in a large group of markers likely to be relevant, in combination with other markers, in the diagnosis or prognosis of inflammations and infections, including sespsis.

### SUMMARY OF THE INVENTION

Recently, the inventors found a close correlation between the evolution of S100A8 and A9 gene expression and the plasma level of the S100A8/A9 complex during the recovery phase of human septic shock (Payen et al., 2008).

The experimental part below reports the results of a further investigation in patients with septic shock examining gene expression of S100 A8 and A9 and plasma levels of the S100A8/A9 complex at day 0 after occurrence of the second organ failure. In this multicentre study, the inventors found a large difference in the plasma S100A8/A9 complex level between eventual survivors and non-survivors, with a specificity and sensitivity of 100%. The threshold for risk of death was determined as 8.1 µg/ml.

### BRIEF DESCRIPTION OF THE DRAWINGS

**Figure 1****:** Box and whisper plot of the plasma level of the S100 A8/A9 complex in survivors (white box) and non-survivors (black box) of septic shock on days 0 and 1 after developing their second organ failure. Statistics were performed using the Mann Whitney non-parametric test.
**Figure 2****:** Box and whisper plot of S100 A8 (left) and A9 (right) gene expression measured by RT-PCR in circulating white cells in survivors (white box) and non-survivors (black box). Statistics were performed using the Mann Whitney non-parametric test.
**Figure 3****:** Correlation between S100A8 and S100A9 gene expression measured by RT-PCR. Statistics were performed using the Spearman test.
**Figure 4****:** Receiver operator characteristic (ROC) curve for 28-day mortality prediction in global population (n = 111). Area under the curve: 0.99.
**Figure 5****:** Trend over time of S100A8/A9 complex, according to outcome, in plasma. Results expressed in µg/ml. Effectives in brackets. * : p ≤ 0.0001 with the Mann Whitney test.
**Figure 6****:** Trend over time of S100A8 gene expression, according to outcome, in circulating mononuclear cells. Results expressed in units of fluorescence. Probe set ID on Affymetrix HG-U133 Plus 2.0 array: 202917_s_at. Effectives in brackets.
**Figure 7****:** Trend over time of S100A9 gene expression, according to outcome, in circulating mononuclear cells. Results expressed in units of fluorescence. Probe set ID on Affymetrix HG-U133 Plus 2.0 array: 203535_at. Effectives in brackets.
**Figure 8****:** S100A8/A9 in patients without shock at D0.
   N=8 survivors
   N=8 non survivors

### DETAILED DESCRIPTION OF THE PREFERRED EMBODIMENTS

In the present text, the terms "sepsis", "severe sepsis" and "septic shock" are to be understood according to the definitions established in 1991 by the American College of Chest Physicians (ACCP) and the Society of Critical Care Medicine (SCCM) and confirmed in 2001 (Levy et al., 2003). These and other definitions used herein are summarized below:
- A "sepsis" is a systemic inflammation in response to infection.
- A "severe sepsis" is defined as a sepsis with at least one organ dysfunction.
- Among severe sepsis syndromes, the most severe cases exhibit two organ failures or even more. Although not very common, some severe sepsis cases with two organ dysfunctions distinct from acute circulatory failure are observed: this kind of cases are defined as "severe sepsis with at least two organ failures", and are distinct from septic shocks.
- A "septic shock" is defined as a sepsis with acute circulatory failure.
- An "acute circulatory failure" is a persistent arterial hypotension (systolic arterial pressure<90 mm Hg, a MAP<60 mmHg or a reduction in systolic blood pressure of >40 mm Hg from baseline) despite adequate volume resuscitation, in the absence of other causes for hypotension.
- A "septic shock with at least two organ failures" is a septic shock with at least one organ failure (e.g., kidney, liver, ...) in addition to the acute circulatory failure.
- In the present text, patients who are considered are those, either in severe sepsis or in septic shock, but in both cases with at least two organ failures. For these patients, "day 0" designates the 24-hours period after the onset of the second organ failure.

As recalled above, sepsis is the consequence of an abnormal presence of micro-organisms. Non-limitative examples of micro-organisms which can provoke sepsis are:

| | | |
|---|---|---|
| 1- | Bacteria: | |
| | gram positive bacilli: | *Corynebacterium* |
| | | *Clostridium* |
| | | *Listeria* |
| | | *Bacillus* |
| | gram negative cocci: | *Neisseria* |
| | gram negative bacilli: | enterobacteria: *E*. *coli, Shigella, Klebsiella, Salmonella, Serratia, Proteus, Pseudomonas, Yersinia* |
| | | *Rickettsia* |
| | | *Haemophilus* |
| | | *Legionella* |
| | | *Providencia* |
| | | *Bacteroides* |
| | gram positive cocci: | *Streptococcus* |
| | | *Staphylococcus* |
| 2- | Viruses: | Herpes viruses (*Herpes viridae*) : Herpes simplex, cytomegalovirus, varicella, zona... |
| | | *Influenzae* virus |
| | | Paramyxovirus |
| | | Coronavirus |
| | | *Parainfluenzae* virus |
| | | Respiratory syncytial virus |
| | | Arbovirus |
| 3- | Parasites: | *Plasmodium* |
| | | *Toxoplasma* |

Other definitions will be specified below, when necessary.

A first aspect of the present invention is a method for *in vitro* establishing a prognosis for a subject in severe sepsis with at least two organ failures, or for a subject in septic shock with at least two organ failures, comprising the following steps:
(i) from a plasma sample from said subject, measuring the level of S100A8 and/or S100A9 and/or the S100A8/A9 complex,
(ii) comparing said level to a predetermined threshold,
wherein a level of S100A8 and/or S100A9 and/or the S100A8/A9 complex above said predetermined threshold is indicative of a bad prognosis and a level of S100A8 and/or S100A9 and/or the S100A8/A9 complex below said predetermined threshold is indicative of a good prognosis.

Of course, depending on the species which is measured (S100A8 and/or S100A9 and/or the S100A8/A9 complex), the threshold will be predetermined for the same. In a preferred embodiment, the level of S100A8/A9 complex is measured, either specifically or in combination with free S100A8. Alternatively, the level of total S100A9 (free and in the S100A8/A9 complex), is measured. In what follows, "S100A8/A9" will be used to designate a species selected in the group of S100A8, S100A9, the S100A8/A9 complex or a combination thereof.

According to a particular embodiment of this method, the biological sample used in step (i) has been collected at day 0, day 1 or day 2 after the onset of the second organ failure, so that an early prognosis can be established. In a preferred embodiment for establishing an early prognosis, the biological sample has been collected at day 0. However, the inventors have demonstrated that S100A8/A9 remains a reliable prognostic at any date during the four weeks following the onset of the second organ failure (Example 2). Hence, the present method can also be performed with a sample collected at any date between day 2 and day 28 after the onset of the second organ failure.

The biological sample is plasma.

The threshold to be considered when performing the above method is predetermined by measuring the level of S100A8/A9 in a representative cohort of individuals having undergone a severe sepsis or septic shock with at least two organ failures, and for whom the outcome is known. The threshold is calculated to obtain the best predictability (sensitivity and specificity) for the risk of death. For example, when the level of S100A8/A9 is measured in the plasma with a technology similar to that described in the experimental part, a predetermined threshold of 7 to 9 µg/ml, preferably from 7.8 to 8.3 µg/ml and for example 8.1 µg/ml, can be considered. On the first cohort used in the present study, the level of S100A8/A9 complex led to a sensitivity and a specificity of prognosis (risk of death) at D0 and D1 of 100%, considering this threshold (Example 1). The relevancy of this threshold was then confirmed for establishing a prognosis later after the onset of the second organ failure (Example 2). Of course, the skilled artisan is free to re-evaluate this threshold on a larger cohort of patients, and by using any kind of technology for measuring S100A8/A9 level. The skilled artisan can also refine the threshold for particular subpopulations, depending on the type of sepsis (number and type of organ failures, sepsis source (lung, abdomen, ...), causative pathogens, ...), or any other parameter regarding the patients or their pathologies (age of patient, associated treatment, particularly immunosuppressive drugs, type of comorbities, ...). Indeed, data obtained in a small cohort of patients in severe sepsis without shock (see Example 4 below) suggest that an appropriate threshold for performing the method according to the invention for this type of patients may be slightly superior to 8.1 µg/l, for example between 8.4 and 9, 9.5 or 10µg/l.

In a particular embodiment, the measure performed in step (i) is done by an immunoassay, for example with an antibody which specifically binds to the S100A8/A9 complex. Several examples of antibodies specifically binding to S100A8/A9 complex (also referred to as MRP8/14 complex) have already been described in the literature, for example by Ikemoto et al. (Ikemoto et al., 2003). Of course, alternative techniques can be used to quantify the S100A8/A9 complex in said biological sample, such as, for example, the technique described by Roth et al. (Roth et al., 1993). The skilled artisan can also use, instead of antibodies specific for the S100A8/A9 complex, any other molecule specifically binding to said complex, such as, for example, antibody fragments or specifically designed aptamers. Aptamers are single stranded nucleic acid molecules (DNA or RNA) that are selected *in vitro* for their ability to bind to a target molecule; this selection can be performed, for example, by the SELEX method (Systematic Evolution of Ligands by Exponential Enrichment) described in US 5,270,163. Besides, the crystal structure of the human S100A8/A9 complex can be used by the skilled artisan for obtaining molecules specifically binding to said complex (Korndorfer et al., 2007). Antibodies or other binding molecules recognizing free S100A8 or free S100A9 in addition to the S100A8/A9 complex, or specifically recognizing the free form of S100A8 or S100A9 can also be used in the context of the present invention, provided they also enable the determination of a threshold for discriminating the patients according to their outcome.

A particular immunoassay which can be used to perform a method according to the present invention is an ELISA assay such as described in the experimental part below. Alternatively, fluorescently labeled antibodies can be used, for example for performing flux cytometry. Of course, the skilled artisan can choose any other immunoassay for performing a method according to the present invention.

Alternatively, techniques different from immunoassays can be used to perform the methods according to the invention. In particular, mass spectrometry can advantageously be used to that purpose.

If necessary, the skilled artisan can combine several markers for establishing a prognosis in cases of sepsis or septic shock with at least two organ failures. Amongst the markers which can be used in combination with S100A8/A9 concentration, procalcitonin (PCT), N-terminal pro-brain natriuretic peptide (BNP), soluble triggering receptor expressed on myeloid cells-1 (sTREM), IL-6 and sRAGE can be cited, as well as SAPS II score, *etc.* The present invention hence also pertains to a method as described above, further comprising a step of measuring the level of at least one other species in a plasma sample from said patient (the same biological sample as that in which S100A8/A9 concentration is measured, or another biological sample if appropriate), and a step of comparing said level to a predetermined threshold. By "species" is herein understood any component, molecule or complex, which can be used as a marker. In a particular embodiment, said other species is/are measured in the same biological sample as S100A8/A9, and is/are selected amongst procalcitonin (PCT), N-terminal pro-brain natriuretic peptide (BNP), soluble triggering receptor expressed on myeloid cells-1 (sTREM), IL-6 and sRAGE.

Also disclosed is a method for performing a follow-up of a patient in severe sepsis with at least two organ failures or in septic shock with at least two organ failures, by measuring the evolution of the plasma level of S100A8/A9 in said patient, wherein a decrease in said level indicates that said patient is recovering. According to this method, if a patient had a level of S100A8/A9 at D0 above the predetermined threshold defined above, and if said level remains above the threshold, this indicates that the patient has a great probability of death.

Also disclosed is a method for performing a follow-up of a patient in severe sepsis or in septic shock with at least two organ failures, by measuring the evolution of the expression level of S100A8 and/or S100A9 in said patient, wherein a decrease in said level indicates that said patient is recovering. This method is illustrated by the publication of D. Payen *et al*., (Payen et al., 2008). When performing this method, the expression level of S100A8 and/or S100A9 will preferably be measured by quantitative amplification, for example by quantitative RT-PCR as described in Payen *et al., supra.*

In the above-described follow-up methods, the measures (of the S100A8/A9 complex or of the expression of S100A8 or S100A9) are performed on biological samples obtained from said patient at several time points after admission, for example each day during the first week and then, depending on the clinical context, at the same frequency or at a lower frequency.

Also disclosed is a method for helping decision for treatment withdrawal for a patient in severe sepsis with at least two organ failures or in septic shock with at least two organ failures, comprising the following steps:
(i) establishing a prognosis for said patient, by a method according to claim 1 (as soon as possible after the onset of the second organ failure);
(ii) measuring the level of S100A8/A9 in a biological sample from said patient, obtained after several days (*e.g.,* 2 weeks) of treatment;
wherein if no decrease in the level of S100A8/A9 is observed and if the clinical status remains severe, treatment withdrawal is decided. When performing this method, the physician will consider that the clinical status remains severe if the patient still has two organ failures or more. Treatment withdrawal will in particular be decided if the level of S100A8/A9 measured in step (i) was above the above-defined threshold and remains above this threshold after several days of treatment.

Since the present invention provides a reliable prognosis marker for patients in very severe conditions (i.e., in severe sepsis with at least two organ failures or in septic shock with at least two organ failures), this prognosis marker can be used to better select the individuals to be enrolled in clinical trials for testing new treatments aiming at improving either the duration of intensive support before the patient leaves the intensive care unit or the outcome of these pathologies.

In the first case, the patients who will be enrolled are those with a good prognosis, in order to avoid noise related to "desperate" patients. The invention hence also pertains to a method for determining if a subject in a very severe condition with at least two organ failures is to be enrolled in a clinical trial for evaluating the efficiency of a pharmaceutical treatment for shortening the need of intensive support for such patient, wherein said method comprises a step of establishing a prognosis for said subject by a method as described above, and wherein said patient is enrolled if the measured level of S100A8/A9 is below the predetermined threshold.

To the contrary, patients with a bad prognosis will be enrolled in trials for evaluating new treatments for improving outcome of very severe conditions with at least two organ failures, so that a drug with potential severe side-effects will not be given to patients supposed to recover by "classical" resuscitation, and so that the results be free of noise related to patients who would have recovered without this new drug or treatment. Hence, the present invention also relates to a method for determining if a subject in severe sepsis with at least two organ failures or in septic shock with at least two organ failures is to be enrolled in a clinical trial for evaluating the efficiency of a pharmaceutical treatment for improving outcome for such a patient, comprising a step of establishing a prognosis for said subject by a method as above-described, wherein said subject is enrolled if the measured level of S100A8/A9 is above the predetermined threshold.

As a corollary of the above method, the invention also pertains to a method for testing the efficiency of a pharmaceutical treatment for improving outcome of severe syndromes with at least two organ failures, comprising the following steps:
(i) selecting a patient in severe sepsis with at least two organ failures or in septic shock with at least two organ failures, and determining the level of S100A8/A9 in a plasma sample from said patient obtained before the beginning of said pharmaceutical treatment;
(ii) from at least another sample from said patient, obtained after the beginning of said pharmaceutical treatment, determining the level of S100A8/A9;
(iii) comparing said obtained values;
wherein a decrease of S100A8/A9 level following the beginning of the pharmaceutical treatment indicates that said treatment has been beneficial to the patient and is likely to improve outcome of severe syndromes with at least two organ failures.

In a preferred embodiment of the above-method, step (i) is performed at day 0 after the onset of the second organ failure, and the selected patient preferably has a S100A8/A9 level above a predetermined threshold as defined above. In this latter case, a new innovative treatment will be considered as beneficial to the patient and most likely to improve outcome of severe syndromes with at least two organ failures if the S100A8/A9 complex level decreases below said threshold.

The skilled artisan can adapt this method for performing it with a follow-up of the patient based on the measure of S100A8 and/or S100A9 expression level instead of (or in addition to) the level of S100A8/A9 complex. As described in Example 3 below, S100A9 expression level is more appropriate than S100A8 expression level to this purpose.

Another aspect of the present invention is a kit for performing any of the above-described methods based on the measure of the S100A8/A9, comprising a molecule specifically binding to the S100A8/A9 complex and/or specifically binding to S100A8 or S100A9 (either specifically in their free form or in both their free form and in the S100A8/A9 complex), and a notice of use explaining the predictive value of the plasma level of S100A8/A9 on the outcome of severe sepsis and septic shock (especially with two organ failures). In a preferred embodiment of the kit, said molecule binds to the S100A8/A9 complex. The skilled artisan can choose any kind of specific binder, such as an antibody, and antibody fragment, an aptamer, a sugar *etc.,* to put into such a kit, provided this binder can be used to measure the level of S100A8/A9 in an appropriate biological sample from a patient. In a preferred embodiment, the notice also provides a protocol description for measuring the S100A8/A9 level in a plasma sample as well as an indication of the threshold above which the level will be indicative of a bad prognosis for a patient. In an even more preferred embodiment, information concerning the sensitivity and specificity of the test which can be carried out with the kit will also be indicated in the notice, in relation with the threshold.

Of course, the kit according to the invention can also comprise other components selected amongst reagents for performing an immunoassay (buffers, enzymes, labeling molecules, *etc.*), quality controls, one or more calibrator(s), *etc..*

Other characteristics of the invention will also become apparent in the course of the description which follows of the biological assays which have been performed in the framework of the invention and which provide it with the required experimental support, without limiting its scope.

### EXAMPLES

### Example 1: Multicentre evaluation of plasma S100A8/A9 complex as an early prognostic marker in septic shock

### Materials and Methods

All the experiments reported below have been carried out with the following materials and methods.

### Patients

This multi-centre study was approved by the Cochin Hospital Ethics Committee (# CCPPRB 2061) and involved patients from four Intensive Care Units (two medical, two surgical). Only patients fulfilling the criteria of septic shock defined according to the ACCP/SCCM consensus conference (Bone et al., 1992) were screened. Inclusion criteria required patients in septic shock to have at least two organ failures as defined by the SOFA (Sequential Organ Failure Assessment) score (Vincent et al., 1998). The first blood sample was taken on day 0 (DO), defined as occurring within 24 hours of development of the second organ failure.

The training cohort enrolled patients fulfilling the inclusion criteria from whom blood samples were taken for gene expression and for plasma levels of the S100A8/A9 complex. The population was sized to fit the calculated statistical power. A second sample was taken on D1 to test the stability of the marker.

Samples from the testing cohort after permission of ethical committee were obtained from patients fulfilling the same inclusion criteria who were enrolled into a multicentre study on severe sepsis (Programme Hospitalier de Recherche Clinique n° AOR 02006) during the same period (February 2004 to November 2005). This cohort was used to validate the predictive ability of the plasma complex S100A8/A9 level taken at day 0 from the training cohort.

### Flow cytometry for monocyte HLA-DR expression measurement:

Whole blood was incubated with appropriate antibodies conjugated with fluorochrome (Fluorescein isothiocyanate (FiTC) or Phycoerythrin (PE)): anti CD14-FiTC (clone RMO52, Beckman Coulter, Marseille, France) and PE conjugated irrelevant isotype control antibodies (Simultest control, BD Biosciences, San Jose, CA, USA) or anti HLA-DR-PE (clone L243, BD Biosciences). Conversion of mean fluorescence intensities into the number of Antibody Bound per Cell (AB/C) was performed using QFCM (Quantitative Flow CytoMetry).

### Plasma cytokines measurements:

Plasma IL-10 (optEIA™ set; PharMingen, San Diego, CA, USA), IL-12p40 and MIF (R&D Systems, Abingdon, Oxon, UK) concentrations were determined by an immunoenzymatic method (ELISA) according to the manufacturer's instructions. Standard samples ranged from 7.8 to 500 pg/ml for IL-10, and from 31.2 to 2000 pg/ml for IL-12p40 and MIF. Detection thresholds were 2.7 ± 3.1 pg/ml for IL-10, 25.8 ±33.3 pg/ml for IL-12p40, and 25.7 ± 34.6 pg/ml for MIF.

### Plasma S100A8/A9 complex level

The technique was adapted from a previous publication (Ikemoto et al., 2003) using blood taken from 34 healthy subjects, paired by age and sex. Levels averaged 0.26 µg/ml with a range of 0.052-0.468 µg/ml. Briefly, 100 µl of diluent solution were added to each well of a 96-well polycarbonate plate coated with the first antibody (Mo2B9; 0.166 mg/L). Plasma samples, previously diluted with a working Block-Ace™ (Dainippon Pharmacology Co Ltd) solution or the MRP8/14 complex calibrator solution, were then added and mixed for 15 seconds. The plate was then incubated for 1 hr to allow the immunological reaction to proceed. After five washings, 100 µl of F(ab')2-biotin conjugate (second antibody Mo3D2 in the working Block-Ace™ solution containing 0.5 g/L thimerosa) was added and the plate incubated for a further hour. After repeat washing as above, 100µl of streptavidin-HRP conjugate diluted 1500-fold with the working Block-Ace solution was added and the plate incubated for a further 30 min. HRP activity was determined by colorimetry. Within-run coefficient of variations (CVs) were 3.9-5.6%, between-day CVs were 5.9-7.6%, and mean recovery was 98% (range 85-103%). The assay was performed by an investigator blinded to the clinical outcomes.

### S100A8-A9 gene expression by quantitative RT-PCR

RNA samples were obtained at D0 and D1 after elimination of mature PMNs by Ficoll gradient centrifugation. Cell analysis included morphological characteristics by light scatter and CD66b expression (FACScalibur) for evaluating the proportion of immature cells (granulocyte lineage) (Payen et al., 2008). At D0 and D1, the proportion of peripheral blood mononuclear cells (PBMC) was approximately 50%. Total RNA was extracted using the Rneasy kit (Qiagen) and samples were treated with Rnase-free DNAse I. Quality and quantity were estimated on an Agilent bioanalyzer and quantities of total RNA were confirmed on a Nanodrop spectrophotometer. qRT-PCR was performed for S100A8 (Hs00374264_gl) and S100A9 (Hs00610058_ml) according to the manufacturer's specifications (Applied Biosystems, Foster City, CA, USA, Table 1) and compared to an endogenous control eukaryotic 18S rRNA (Hs99999901_s1). Results are expressed in Delta Ct (concentration threshold): CtS100Ax-Ct18S.

**Table 1: Characteristics of Gene expression Assays for Real-Time PCR. Assays are designed and distributed by Applied Biosystems**

| Gene Symbol | Gene Name | NCBI Gene ref | Assay ID |
|---|---|---|---|
| 18S | Eukaryotic 18S rRNA | X03205.1 | Hs99999901_s1 |
| S100A8, MRP8 | S100 calcium binding protein A8 | NM_002964.3 | Hs00374264_g1 |
| S100A9, MRP14 | S100 calcium binding protein A9 | NM_002965.3 | Hs00610058_m1 |

### Statistics

Quantitative results are expressed as the median and 25^{th}-75^{th} percentiles. Comparison of quantitative variables was performed using a Mann-Whitney test, and qualitative variables by chi-squared testing. Correlation studies were performed by the Spearman correlation test. A p value <0·05 was considered statistically significant. Based on data previously published in human septic shock (Payen et al., 2008), the inventors assumed a mortality of 40% in septic shock patients (Annane et al., 2007; Sprung et al., 2008) and a difference in S100A8/A9 complex between survivors and non-survivors of 2 µg /ml, with a standard error of 2·35 (Payen et al., 2008). Accordingly, a sample size of 47 patients needed to be recruited for the training cohort, controlling for a type I error probability of 0.05 and a power of 0.80.

The prognostic performance of the plasma level of S100A8/A9 complex was assessed through computation of an area under the ROC curve (AUC). Because of the observed sensitivity and specificity values in the training cohort, and to account for the relatively small sample size, the inventors provided estimates of sensitivity and specificity based on the Laplace method (Agresti and Coull, 1998). To validate these results, a second independent cohort (testing cohort) of patients was studied using the same entry criteria. The main characteristics of the two cohorts were compared, and prognostic analyses of the S100A8/A9 complex performed, including estimation of the AUC as well as sensitivity and specificity values based on the training S100A8/A9 threshold.

Statistical analyses were performed on SAS 9.1 (SAS Inc, Cary, NC) and R 2.8.0 (http://www.R-project.org) software packages.

### Results

### Training cohort

Forty nine patients were enrolled; their clinical characteristics are summarized in Table 2 below. Surviving Sepsis Campaign guidelines for patient management were followed (Dellinger et al., 2004). Nine patients (18%) received activated Protein C while 35 (71%) were treated with hydrocortisone. Thirty one patients had undergone a surgical procedure within the previous two days. Fifteen patients died within the 28-day study period, 12 during the first 7 days. The three patients who died after D7 (one at D24 and two at D26) did so because of non-treatable respiratory failure and severe hypoxaemia, delayed multiple organ failure, and a moribund condition mandating treatment limitation. At D0, the SAPSII clinical severity score differed between survivors and non-survivors, whereas the SOFA score was similar (Table 2).

**Table 2: Clinical characteristics, type of sepsis, and cardiovascular support in the training cohort at D0**

| Median (25^{th}-75^{th} percentiles) | Total n=49 | survivors n=34 | Non-survivors n=15 | P |
|---|---|---|---|---|
| Age (y/o) | 64 (51-75) | 62 (50-68) | 75 (67-84) | 0.0040 |
| Sex M/F | 34/15 | 24/10 | 10/5 | NS |
| SAPSII | 57 (43-63) | 52 (42-60) | 70 (62-81) | <0.0001 |
| SOFA D0 | 10(8-12) | 10(8-11) | 11 (9-13) | NS |
| number of organ failures | 3(3-4) | 3(3-4) | 4 (3-5) | 0.0215 |
| Postoperative | 31/18 | 21/13 | 10/5 | NS |
| Infection site: | | | | |
| thorax | 10 | 7 | 3 | |
| abdomen | 24 | 15 | 9 | |
| urinary tract | 7 | 6 | 1 | |
| CNS | 1 | 0 | 1 | |
| peripheral | 7 | 6 | 1 | |
| Therapies: | | | | |
| Catecholamines (µg/kg/min): | | | | |
| • Norepinephrine (n=46) | 0·4 (0·2-0·6) | 0·3 (0·1-0·5) | 0·6 (0·4-1·3) | 0·0051 |
| | 0·2 (0·1-0·9) | 0·4 (0·1-0·8) | 0·2 (0·1-1·0) | NS |
| • Epinephrine (n=9) | 10·0 (6·3-18·8) | 5 (5-5) | 15 (10-20) | 0·0528 |
| • Dobutamine (n=7) | 10(0) | 10(0) | - | - |
| • Dopamine (n=1) | 35 | 21 | 14 | |
| Hydrocortisone treated (n) | 9 | 4 | 5 | |
| Activated protein C treated (n) | | | | |

Blood inflammatory mediator results are summarized in Table 3. There was no difference at D0 between survivors and non-survivors for either plasma levels of IL-12p40, or HLA-DR monocyte expression. Plasma IL-10 and MIF levels were significantly higher in patients who eventually died (p< 0.05 and p< 0.01, respectively), however there was a large overlap in values between the two groups.

**Table 3: Plasma cytokine and MIF levels, and monocyte expression of HLA-DR at D0 in the training cohort. MIF: macrophage migration inhibiting factor.**

| Median (25^{th}-75^{th} percentiles) | Total n=49 | Survivors n=34 | Non-survivors n=15 | p |
|---|---|---|---|---|
| HLA-DR (AB/C) | 2797 (1441-4538) | 2826 (1410-5093) | 2762 (1485-4307) | NS |
| IL-10 (pg/ml) | 150 (66-478) | 128 (40-269) | 326 (133-653) | 0.0156 |
| IL-12p40 (pg/ml) | 15(0-43) | 20 (0-50) | 12 (0-34) | NS |
| MIF (pg/ml) | 2118 (955-9163) | 1460 (713-3746) | 9049 (2795-20284) | 0.0055 |

### S100A8/S100A9 complex plasma protein and mRNA levels

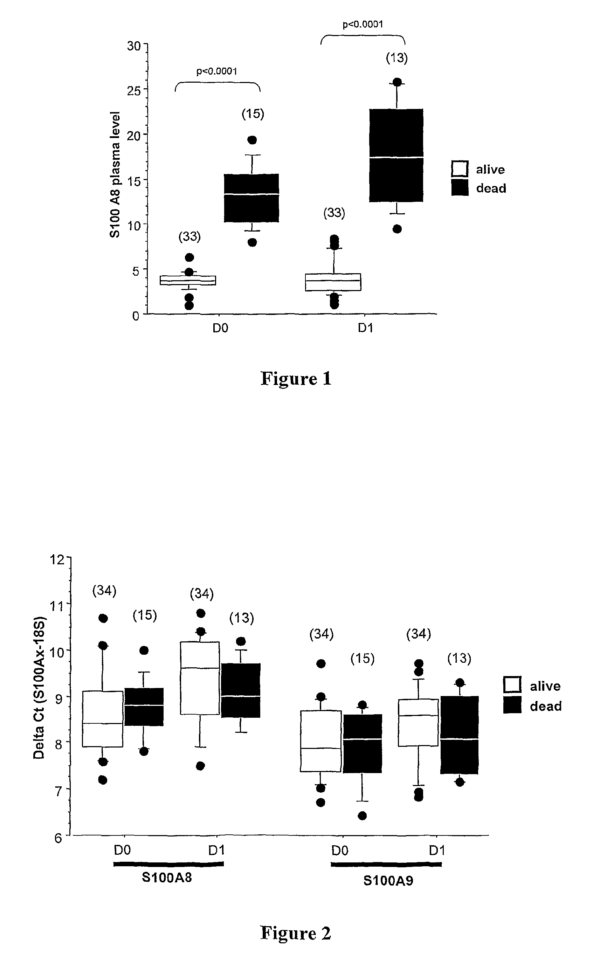

At D0, plasma levels of the S100 A8/A9 complex differed markedly between eventual survivors and non-survivors (3.7 µg/ml (3.2-4.2) *vs* 13.3 µg/ml (10.1-15.6), p< 0.0001). There was no overlap between the groups. Similar results were observed on D1 (Figure 1). As a consequence, specificity and sensitivity of 100% were obtained, with a threshold plasma level for mortality risk of 8.1 µg/ml. Laplace estimate of sensitivity was 94.1% (95% confidence interval: 84.7%-100%) for a specificity of 97.2% (95% confidence interval: 92.6%-100%). Importantly, this was not influenced by the number of organ failures measured on D0. Similar plasma levels were seen in those with 2 organ failures [3.5 µg/ml IQ 1.4 (n=8)] or >2 organ failures [3.7 µg/ml IQ 0.9 (n=25)].

Of the nine patients (18%) receiving aPC after D0, five survived (D0 levels: 3.4 µg/ml; IQR 1.1) and 4 died (D0 levels: 15.7 µg/ml; IQR 3.6). Likewise, D0 levels in patients treated subsequently with corticosteroids (35 patients) were 3.7 µg /ml (IQR 1.0) in survivors *vs* 13.8 µg /ml (IQR 5.6) in eventual non-survivors. The dosage requirement of catecholamines (norepinephrine and dobutamine) was higher in eventual non- survivors (p<0.01) (Table 1).

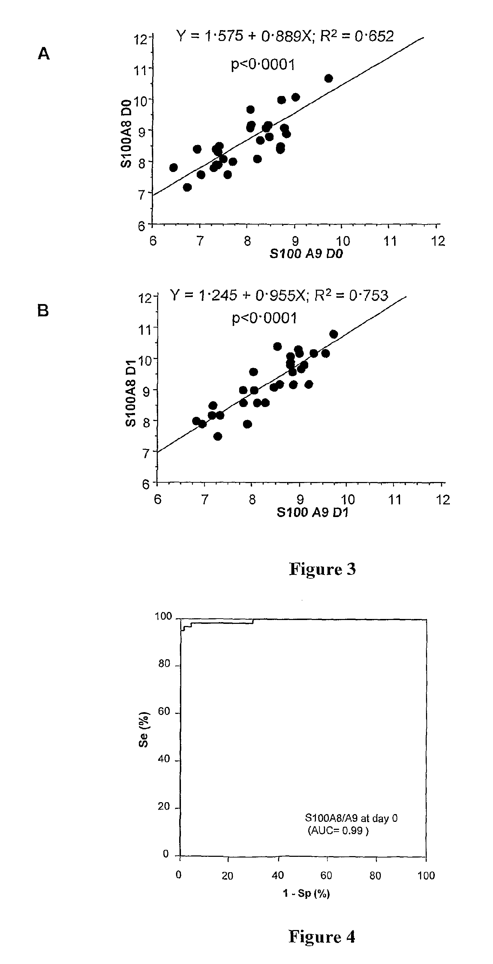

The mRNA levels for S100A8 or S100A9 genes in PBMC and immature cells did not differ between survivors and non-survivors on either D0 or D1 (Figures 2A and B). Gene expression for S100A8 and S100A9 correlated well (r=0.81 at D0 and r=0.87 at D1, p<0.0001 Figure 3), however no correlation was seen with plasma S100A8/A9 complex levels on either D0 or D1 (data not shown).

### Testing cohort

Samples from 62 patients fulfilling the same entry criteria were studied. Clinical characteristics and inflammatory variables according to outcome are depicted in Tables 4 and 5. Clinical variables were comparable with the training cohort, except for mortality by day 28 (Table 6) which was significantly higher (56.5% vs. 30.6%; p= 0.01). In the testing cohort, HLA-DR expression and plasma MIF levels were similar, but the IL-10 value was lower (p<0.05). As with the first cohort, there was a clear difference in plasma levels of S100A8/A9 complex between eventual survivors [5.3 µg/ml (4.43-6.43)] and non-survivors [12.5 µg/ml (10.23-14.08) p <0.0001]. The AUC value was 0.99 and the 8.1 µg/ml threshold plasma value obtained from the training cohort distinguished survivors from non-survivors with a sensitivity and specificity of 94.3% and 100% (Laplace estimate: 96.55%), respectively. Only two patients were misclassified; their values of plasma S100A8/A9 suggested a good prognosis yet they died after three weeks with decompensated hepatic failure on the background of liver cirrhosis. As in the training cohort, neither the number of organ failures nor treatment with aPC or hydrocortisone influenced the prognostication provided by the plasma S100A8/A9 level (data not shown).

Pooling of the two cohorts gave a population of 111 patients with an homogenous severity at D0. The calculated threshold of plasma level of S100A8/A9 to predict outcome in this global population was identical to the one calculated from the training cohort (8.1 µg/ml).

**Table 4: Clinical characteristics, type of sepsis, and cardiovascular support in the Testing cohort at D0**

| Median (25^{th}-75^{tn} percentiles) | Total n=62 | Survivors n=27 | Non-survivors n=35 | P-value |
|---|---|---|---|---|
| Age (y/o) | 63 (53-77) | 67 (57-78) | 59 (50-69) | NS |
| Sex M/F | 36/26 | 13/14 | 23/12 | NS |
| SAPSII | 51 (42-64) | 50 (41-59) | 53 (43-67) | NS |
| SOFA D0 | 10(8-12) | 9(8-12) | 10 (9-13) | NS |
| No Organ Failures | 3 (3-4) | 3 (3-4) | 3 (3-4) | NS |
| Postoperative | 17/45 | 10/17 | 7/28 | NS |
| Infection site: | | | | |
| • thorax | 33 | 15 | 18 | |
| • abdomen | 11 | 3 | 8 | |
| • riunary tract | 4 | 2 | 2 | |
| • CNS | 3 | 1 | 2 | |
| • peripheral | 11 | 6 | 5 | |
| Therapies: | | | | |
| Catecholamines (µg/kg/min): | 0·45 (0·27-1·04) | 0·7 (0·31-0·92) | 0·43 (0·24-1·20) | |
| • Norepinephrine (n=52) | 0·48 (0·23-1·40) | | 0·78 (0·20-1·60) | NS |
| | 10 (5-20) | 0·4 (0·32-0·57) | 10 (7·5-20) | NS |
| • Epinephrine (n=19) | 12(10-20) | | 20 (14-20) | NS |
| | 42 | 10 (4-10·5) | 23 | NS |
| • Dobutamine (n=20) | 5 | 10(9-15) | 4 | |
| | | 19 | | |
| • Dopamine (n=7) | | 1 | | |
| Hydrocortisone (n) | | | | |
| Activated protein C (n) | | | | |

**Table 5: Plasma cytokine levels and monocyte expression of HLA-DR at D0 in Testing cohort. MIF: macrophage migration inhibiting factor**

| Median (25^{th}-75^{th} percentiles) | Total (n=62) | Alive (n=27) | Dead (n=35) | P-value |
|---|---|---|---|---|
| HLA-DR (AB/C) | 2952 (1714-5138) | 2546 (1778-4795) | 3458 (1595-5684) | NS |
| IL-10 (pg/ml) | 85 (16-292) | 42(14-110) | 136 (22-558) | 0.05 |
| IL-12p40 (pg/ml) | 0(0-8) | 0(0-5) | 0 (0-39) | NS |
| MIF (pg/ml) | 2020(1075-5360) | 2287(1099-6104) | 1941 (1006-3945) | NS |

**Table 6: Comparison of the main characteristics of patients from the training and the testing cohorts.**

| Median (25^{th}-75^{th} percentiles) | Training cohort n=49 | Testing cohort n=62 | P |
|---|---|---|---|
| Age (y/o) | 64 (51-75) | 63 (53-77) | NS |
| Sex M/F | 34/15 | 36/26 | NS |
| SAPSII | 57 (43-63) | 51(42-64) | NS |
| SOFA D0 | 10(8-12) | 10(8-12) | NS |
| No Organ Failures | 3 (3-4) | 3(3-4) | NS |
| Postoperative | 31 (63%) | 17 (27%) | |
| Infection site | | | |
| thorax | 10 | 33 | |
| abdomen | 24 | 11 | |
| urinary tract | 7 | 4 | |
| CNS | 1 | 3 | |
| peripheral | 7 | 11 | |
| HLA-DR (AB/C) | 2797 (1441-4538) | 2952 (1714-5138) | NS |
| IL-10 (pg/ml) | 150 (66-478) | 85 (16-292) | 0-0382 |
| MIF (pg/ml) | 2118 (955-9163) | 2020 (1075-5360) | NS |
| Co-morbidities | | | |
| Cancer with metastasis | 10 | 4 | |
| Malignant haemopathy | 0 | 9 | |
| Transplantation | 1 | 4 | |

### Discussion:

We found the plasma level of the S100A8/A9 complex to be an excellent predictor of outcome in patients with septic shock, even when taken as early as the first day of occurrence of the 2^{nd} organ failure. This was independent of the number of organ failures at D0 or the subsequent use of activated Protein C or corticosteroids. It remained stable, at least for the first 2 days, with a predictive threshold for death of 8.1µg/ml. The validity of this threshold obtained in a training cohort of 49 patients was subsequently confirmed in a testing cohort of 62 patients. The plasma protein level difference did not correlate with S100A8 and A9 gene expression on peripheral white blood cells suggesting, at least in this early phase, origins for this complex other than circulating immune cells.

An ideal prognostic marker in septic shock has to be both specific and sensitive for outcome prediction, applicable to a broad population, stable over an adequate time duration for clinical interpretation (1-2 days), easily measured in plasma, exclusively dependent on outcome yet able to be modulated by subsequent effective treatments, and relatively inexpensive. Although limited in size, the above results are highly encouraging as both the training and testing cohorts contained both medical and surgical patients with a calibrated severity at the time of inclusion. Plasma levels in the surviving septic patients corresponded to previous results obtained in patients with acute arthritis of inflammatory aetiologies(de Seny et al., 2008; Foell et al., 2004; Sampson et al., 2002). To our knowledge, similar plasma levels to those found in non-survivors have only been reported during acute rejection of transplanted kidneys (Jung et al., 2008).

The availability of a reliable, early, sensitive and specific prognosticator in septic shock offers several important advantages. These include better selection of patients to enrol in future clinical trials whereby the emphasis in good prognosis patients will be on increasing the speed of resolution and extent of organ failure, whereas mortality improvement (with good long-term quality of life) would constitute the primary goal in predicted non-survivors. For an individual patient, it may avoid futile prolongation of treatment to mitigate unnecessary suffering and to enable more effective utilisation of expensive and limited intensive care resources and therapies. Importantly, in our study, the subsequent use of activated protein C and/or hydrocortisone was not able to influence predicted outcome. This highlights the ongoing debate surrounding these two immunomodulatory agents regarding patient selection, optimal timing and dose of administration (Dellinger et al., 2008). Furthermore, since S100 A8/A9 proteins play a mechanistic role in the pathophysiology of septic shock (Foell et al., 2007; Vogl et al., 2007), new drugs blocking this system may be usefully developed and explored.

The studied population was homogenous in term of severity; eventual survivors and non-survivors could not be distinguished on D0, either clinically or by using plasma markers of inflammation (other than MIF). Though we did not directly compare the prognostic capabilities of plasma S100 A8/A9 protein levels against other recognised biomarkers of sepsis/inflammation, a literature review does suggest superiority. For example, plasma procalcitonin levels were significantly higher in septic non-survivors compared to survivors, with 87.5% sensitivity yet only 45% specificity (Clec'h et al., 2004). The same applies to IL-6, a marker of the severity of systemic inflammation in septic shock (Abraham et al., 2001), HLA-DR expression on circulating monocytes (Monneret et al., 2006), and brain natriuretic peptides measured during the first 3 days of severe sepsis or septic shock (Varpula et al., 2007). Plasma levels of soluble TREM-1 in septic patients (Gibot et al., 2005) had a relatively low sensitivity for outcome prediction, thus precluding its use as a prognostic marker. Recently, Scherpereel *et al.* reported a large overlap in septic survivors and non-survivors of plasma levels of endocan, a circulating proteoglycan (Scherpereel et al., 2006). Plasma sRAGE was also found to be higher in septic non-survivors, with a specificity of 75% and a sensitivity of 84.6% (Bopp et al., 2008).

To date, more than 20 members of the S100 protein family have been described (Ravasi et al., 2004). Three (S100A8, A9, and A12) are specifically linked to innate immune functions. S100A8 and S100A9 are found in granulocytes, monocytes and the early differentiation stages of macrophages (Foell et al., 2007). By contrast, S100A12 (EN-RAGE, extracellular newly identified RAGE binding protein) appears more restricted to granulocytes (Vogl et al., 1999). In a recent study, mice lacking the S100A8-A9 complex were protected from both *Escherichia coli-* and endotoxin-induced lethal shock (Vogl et al., 2007). The S100A8/A9 complex amplified phagocyte responses including intracellular translocation of myeloid differentiation primary response protein (MyD)-88 and activation of interleukin-1 receptor-associated kinase (IRAK)-1 and nuclear factor (NF)-kB, resulting in an elevated expression of tumor necrosis factor-α (TNF-α). As S100A8 specifically interacted with the TLR4-MD2 complex, it represents an endogenous ligand of TLR4.

These phagocyte-specific S100 proteins are actively secreted *via* an alternative pathway bypassing the classical Golgi-route (Rammes et al., 1997). This is typical for DAMP-related factors that have a role in cell homeostasis as intracellular molecules, but turn into proinflammatory danger signals after release to the extracellular compartment due to cell damage, infection or inflammation. This may explain why plasma levels are markedly higher in patients who will not survive, suggesting a more severe loss of cell homeostasis.

Release of S100A8 and S100A9 is independent from *de novo* synthesis and has been associated with down-regulation of mRNA expression of both genes (Foell et al., 2007). The absence of correlation at D0 between the plasma level of the complex S100A8/A9 and white cell S100A8 and A9 gene expression confirms the absence of early *de novo* synthesis.

In conclusion, the discriminative role of the S100A8/A9 complex based on differences detected between survivors and non-survivors in a training cohort, and subsequently validated by a larger testing cohort with a higher mortality rate, strongly supports the utility of this outcome predictor early in the presentation of patients with septic shock with at least two organ failures. Clearly, large-scale testing is required to assess generalisability and the impact of co-morbidities. However, if confirmed, this may open a new era, both in terms of patient management and in targeting a new system for therapeutic modulation.

### Example 2: Trend over time of plasma S100A8/A9 complex level in septic shock patients

The experiments performed as in Example 1, for day 0 and 1, have been completed over 28 days (D0, D1, D7, D14 and D28).

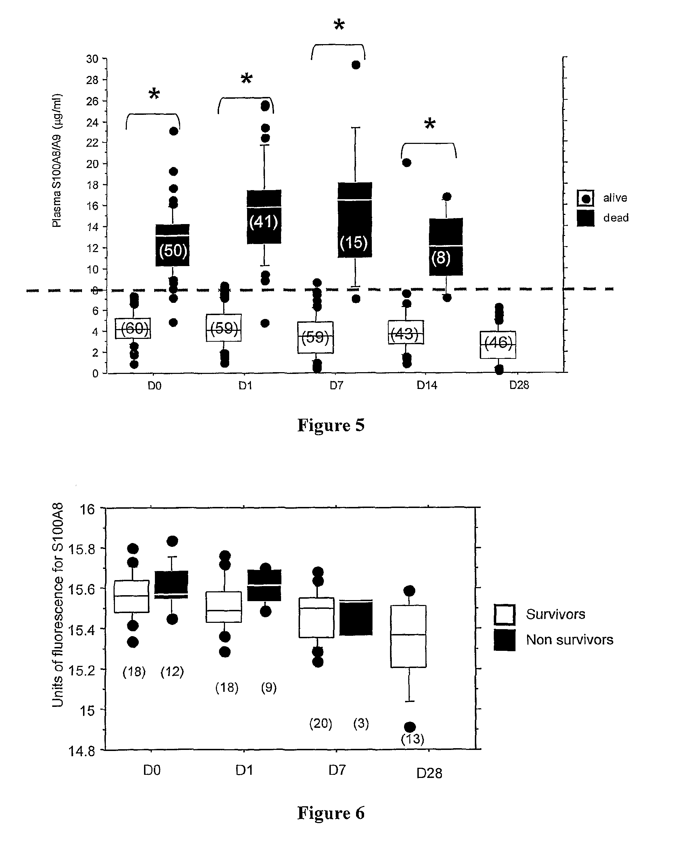

The results on 111 patients (49 of the "training cohort" and 62 of the "testing cohort" of example 1) show that plasma protein level decreased over time in survivors, but not in non-survivors (Figures 5). Plasma S100A8/A9 was still elevated at D28 (median 2.65 µg/ml (IQR 2.60)) in survivors compared to controls.

Only one patient with a final good outcome showed a surprisingly high level of S100A8/A9 complex at D 14. In this patient, S100A8/A9 values were 4.7 µg/ml at D0, 4.0 µg/ml at D1, 4.5 µg/ml at D7, 20.1 µg/ml at D14, although his evolution was uneventful and his final good outcome (discharge before D28). The S100A8/A9 level at D 14 was however not controlled, and this surprising result may be a measure artefact.

The kinetics were studied on both populations using the Friedman test: for the survivors, the S100A8/A9 plasma level decreased:
D0-D28, p<0.0001, n=34
D0-D14, p<0.0001, n=41
D0-D7, p=0.0005, n=57
for patients who died before D28, the S100A8/A9 plasma level increased:
D0-D14, non significant
D0-D7, p=0.0010, n=15.

### Example 3: Trend over time of S100A8 and S100A9 gene expression levels in septic shock patients

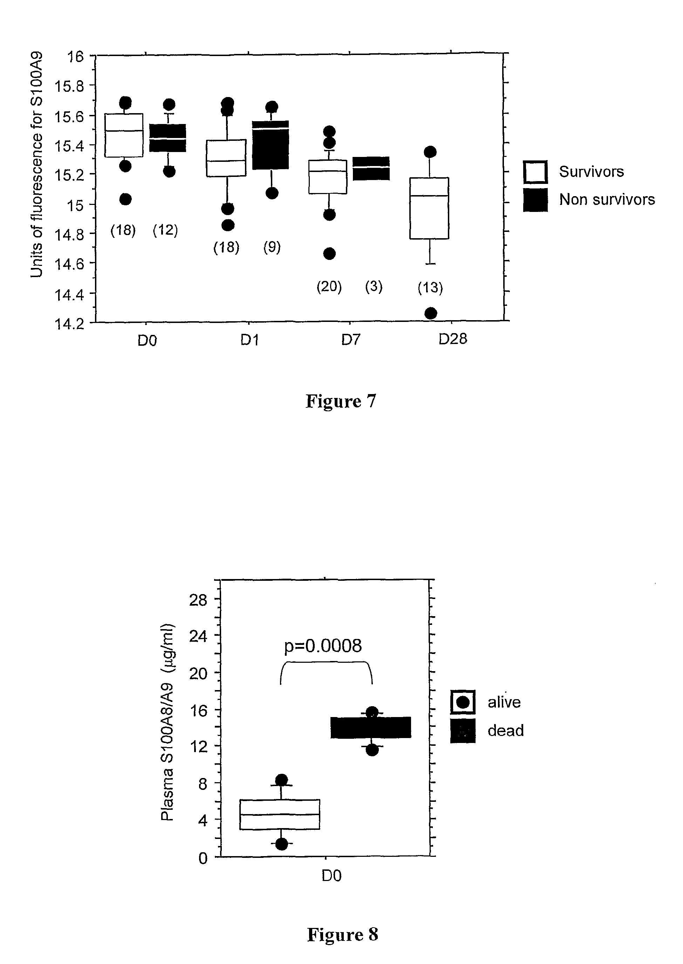

Figure 6 and Figure 7 show the S100A8 and S100A9 gene expressions in 32 patients. These data have been extracted from microarray analysis performed on Affymetrix HG-U133 Plus 2.0 array, using the same methodology as described in EP 2085486 A1.

S100A8 gene expression decreased slightly in survivors between day 0 and day 7, even this was not significant over 28 days. This trend was not observed in non survivors.. The statistics for S100A8 gene expression are as follows:

| | |
|---|---|
| Friedman test in survivors, | D0-D28, n=11: NS |
| | D0-D7, n=18: p=0.0421 |
| Wilcoxon test in survivors, | D0-D1, n=17: NS |
| Wilcoxon test in non-survivors, | D0-D1, n=9: NS |

Mann Whitney test survivors *vs*. non-survivors: significant at D1 p=0.035.

S100A9 gene expression decreased over time in survivors and did not vary in non-survivors. The statistics for S100A9 gene expression are disclosed below:

| | |
|---|---|
| Friedman test in survivors, | D0-D28, n=11: p=0.0199 |
| | D0-D7, n=18: p<0.0001 |
| Wilcoxon test in survivors, | D0-D1, n=17: p=0.0019 |
| Wilcoxon test in non-survivors, | D0-D1, n=9: NS |

Mann Whitney test survivors *vs*. non-survivors: non significant at each time.

### Example 4: Evaluation of plasma S100A8/A9 complex level as an early prognostic marker in severe sepsis without shock

Plasma S100A8/A9 level was measured in 16 patients without shock at D0. These patients were in severe sepsis with two organ failures (same criteria of organ dysfunction as in previous cohort).

Eight died before D28. Plasma S100A8/A9 was significantly higher in non-survivors compared to survivors (Figure 8 and Table 7).

**Table 7 : Descriptive statistics split by status. Mann Whitney test, p=0.0008.**

| | S100_D0, Total | S100_D0, alive | S100_D0, dead |
|---|---|---|---|
| Mean | 9,213 | 4,613 | 13,812 |
| Std. Dev. | 5,095 | 2,307 | 1,391 |
| Std. Error | 1,274 | ,816 | ,492 |
| Count | 16 | 8 | 8 |
| Minimum | 1,400 | 1,400 | 11,600 |
| Maximum | 15,600 | 8,300 | 15,600 |
| # Missing | 0 | 0 | 0 |
| Median | 9,950 | 4,550 | 13,950 |
| IQR | 9,400 | 3,050 | 2,200 |

Only one survivor presented a level (at 8.3 µg/ml) above the proposed threshold of 8.1 µg/ml, suggesting that the threshold for severe sepsis without shock could be slightly superior to the threshold for septic shock. None of the non-survivors presented a level under the threshold of 8.1 µg/ml. A threshold between 8.4 and 9, 9.5 or 10 µg/ml may be appropriate for prognosticating the outcome of patients in severe sepsis without shock.

### REFERENCES

Abraham, E., Laterre, P.F., Garbino, J., Pingleton, S., Butler, T., Dugernier, T., Margolis, B., Kudsk, K., Zimmerli, W., Anderson, P., Reynaert, M., Lew, D., Lesslauer, W., Passe, S., Cooper, P., Burdeska, A., Modi, M., Leighton, A., Salgo, M. and Van der Auwera, P. (2001) Lenercept (p55 tumor necrosis factor receptor fusion protein) in severe sepsis and early septic shock: a randomized, double-blind, placebo-controlled, multicenter phase III trial with 1,342 patients. Crit Care Med, 29, 503-510.
Agresti, A. and Coull, B. (1998) Approximate is better than "exact" for interval estimation of binomial proportions. The American Statistician, 52, 119-126.
Annane, D., Sebille, V., Charpentier, C., Bollaert, P.E., Francois, B., Korach, J.M., Capellier, G., Cohen, Y., Azoulay, E., Troche, G., Chaumet-Riffaud, P. and Bellissant, E. (2002) Effect of treatment with low doses of hydrocortisone and fludrocortisone on mortality in patients with septic shock. Jama, 288, 862-871.
Annane, D., Vignon, P., Renault, A., Bollaert, P.E., Charpentier, C., Martin, C., Troche, G., Ricard, J.D., Nitenberg, G., Papazian, L., Azoulay, E. and Bellissant, E. (2007) Norepinephrine plus dobutamine versus epinephrine alone for management of septic shock: a randomised trial. Lancet, 370, 676-684.
Bernard, G.R., Vincent, J.L., Laterre, P.F., LaRosa, S.P., Dhainaut, J.F., Lopez-Rodriguez, A., Steingrub, J.S., Garber, G.E., Helterbrand, J.D., Ely, E.W. and Fisher, C.J., Jr. (2001) Efficacy and safety of recombinant human activated protein C for severe sepsis. N Engl J Med, 344, 699-709.
Bone, R.C., Balk, R.A., Cerra, F.B., Dellinger, R.P., Fein, A.M., Knaus, W.A., Schein, R.M. and Sibbald, W.J. (1992) Definitions for sepsis and organ failure and guidelines for the use of innovative therapies in sepsis. The ACCP/SCCM Consensus Conference Committee. American College of Chest Physicians/Society of Critical Care Medicine. Chest, 101, 1644-1655.
Bopp, C., Hofer, S., Weitz, J., Bierhaus, A., Nawroth, P.P., Martin, E., Buchler, M.W. and Weigand, M.A. (2008) sRAGE is elevated in septic patients and associated with patients outcome. J Surg Res, 147, 79-83.
Boyd, J.H., Kan, B., Roberts, H., Wang, Y. and Walley, K.R. (2008) S100A8 and S100A9 mediate endotoxin-induced cardiomyocyte dysfunction via the receptor for advanced glycation end products. Circ Res, 102, 1239-1246.
Brunn, G.J. and Platt, J.L. (2006) The etiology of sepsis: turned inside out. Trends Mol Med, 12, 10-16.
Clec'h, C., Ferriere, F., Karoubi, P., Fosse, J.P., Cupa, M., Hoang, P. and Cohen, Y. (2004) Diagnostic and prognostic value of procalcitonin in patients with septic shock. Crit Care Med, 32, 1166-1169.
de Seny, D., Fillet, M., Ribbens, C., Maree, R., Meuwis, M.A., Lutteri, L., Chapelle, J.P., Wehenkel, L., Louis, E., Merville, M.P. and Malaise, M. (2008) Monomeric calgranulins measured by SELDI-TOF mass spectrometry and calprotectin measured by ELISA as biomarkers in arthritis. Clin Chem, 54, 1066-1075.
Dellinger, R.P., Carlet, J.M., Masur, H., Gerlach, H., Calandra, T., Cohen, J., Gea-Banacloche, J., Keh, D., Marshall, J.C., Parker, M.M., Ramsay, G., Zimmerman, J.L., Vincent, J.L. and Levy, M.M. (2004) Surviving Sepsis Campaign guidelines for management of severe sepsis and septic shock. Crit Care Med, 32, 858-873.
Dellinger, R.P., Levy, M.M., Carlet, J.M., Bion, J., Parker, M.M., Jaeschke, R., Reinhart, K., Angus, D.C., Brun-Buisson, C., Beale, R., Calandra, T., Dhainaut, J.F., Gerlach, H., Harvey, M., Marini, J.J., Marshall, J., Ranieri, M., Ramsay, G., Sevransky, J., Thompson, B.T., Townsend, S., Vender, J.S., Zimmerman, J.L. and Vincent, J.L. (2008) Surviving Sepsis Campaign: international guidelines for management of severe sepsis and septic shock: 2008. Intensive Care Med, 34, 17-60.
Foell, D., Frosch, M., Sorg, C. and Roth, J. (2004) Phagocyte-specific calcium-binding S100 proteins as clinical laboratory markers of inflammation. Clin Chim Acta, 344, 37-51.
Foell, D., Wittkowski, H., Vogl, T. and Roth, J. (2007) S100 proteins expressed in phagocytes: a novel group of damage-associated molecular pattern molecules. J Leukoc Biol, 81, 28-37**.**
Gibot, S., Cravoisy, A., Kolopp-Sarda, M.N., Bene, M.C., Faure, G., Bollaert, P.E. and Levy, B. (2005) Time-course of sTREM (soluble triggering receptor expressed on myeloid cells)-1, procalcitonin, and C-reactive protein plasma concentrations during sepsis. Crit Care Med, 33, 792-796.
Grigoryev, D.N., Liu, M., Hassoun, H.T., Cheadle, C., Barnes, K.C. and Rabb, H. (2008) The local and systemic inflammatory transcriptome after acute kidney injury. J Am Soc Nephrol, 19, 547-558.
Ikemoto, M., Tanaka, T., Takai, Y., Murayama, H., Tanaka, K. and Fujita, M. (2003) New ELISA system for myeloid-related protein complex (MRP8/14) and its clinical significance as a sensitive marker for inflammatory responses associated with transplant rejection. Clin Chem, 49, 594-600.
Jung, D.Y., Park, J.B., Lee, E.N., Lee, H.A., Joh, J.W., Kwon, C.H., Ki, C.S., Lee, S.Y. and Kim, S.J. (2008) Combined use of myeloid-related protein 8/14 and procalcitonin as diagnostic markers for acute allograft rejection in kidney transplantation recipients. Transpl Immunol, 18, 338-343.
Korndorfer, I.P., Brueckner, F. and Skerra, A. (2007) The crystal structure of the human (S100A8/S100A9)2 heterotetramer, calprotectin, illustrates how conformational changes of interacting alpha-helices can determine specific association of two EF-hand proteins. J Mol Biol, 370, 887-898.
Levy, M.M., Fink, M.P., Marshall, J.C., Abraham, E., Angus, D., Cook, D., Cohen, J., Opal, S.M., Vincent, J.L. and Ramsay, G. (2003) 2001 SCCM/ESICM/ACCP/ATS/SIS International Sepsis Definitions Conference. Crit Care Med, 31, 1250-1256.
Monneret, G., Lepape, A., Voirin, N., Bohe, J., Venet, F., Debard, A.L., Thizy, H., Bienvenu, J., Gueyffier, F. and Vanhems, P. (2006) Persisting low monocyte human leukocyte antigen-DR expression predicts mortality in septic shock. Intensive Care Med, 32, 1175-1183.
Oppenheim, J.J., Tewary, P., de la Rosa, G. and Yang, D. (2007) Alarmins initiate host defense. Adv Exp Med Biol, 601, 185-194.
Payen, D., Lukaszewicz, A.C., Belikova, I., Faivre, V., Gelin, C., Russwurm, S., Launay, J.M. and Sevenet, N. (2008) Gene profiling in human blood leucocytes during recovery from septic shock. Intensive Care Med, 34, 1371-1376.
Rammes, A., Roth, J., Goebeler, M., Klempt, M., Hartmann, M. and Sorg, C. (1997) Myeloid-related protein (MRP) 8 and MRP 14, calcium-binding proteins of the S100 family, are secreted by activated monocytes via a novel, tubulin-dependent pathway. J Biol Chem, 272, 9496-9502.
Ravasi, T., Hsu, K., Goyette, J., Schroder, K., Yang, Z., Rahimi, F., Miranda, L.P., Alewood, P.F., Hume, D.A. and Geczy, C. (2004) Probing the S100 protein family through genomic and functional analysis. Genomics, 84, 10-22.
Roth, J., Burwinkel, F., van den Bos, C., Goebeler, M., Vollmer, E. and Sorg, C. (1993) MRP8 and MRP14, S-100-like proteins associated with myeloid differentiation, are translocated to plasma membrane and intermediate filaments in a calcium-dependent manner. Blood, 82, 1875-1883.
Sampson, B., Fagerhol, M.K., Sunderkotter, C., Golden, B.E., Richmond, P., Klein, N., Kovar, I.Z., Beattie, J.H., Wolska-Kusnierz, B., Saito, Y. and Roth, J. (2002) Hyperzincaemia and hypercalprotectinaemia: a new disorder of zinc metabolism. Lancet, 360, 1742-1745.
Scherpereel, A., Depontieu, F., Grigoriu, B., Cavestri, B., Tsicopoulos, A., Gentina, T., Jourdain, M., Pugin, J., Tonnel, A.B. and Lassalle, P. (2006) Endocan, a new endothelial marker in human sepsis. Crit Care Med, 34, 532-537**.**
Sprung, C.L., Annane, D., Keh, D., Moreno, R., Singer, M., Freivogel, K., Weiss, Y.G., Benbenishty, J., Kalenka, A., Forst, H., Laterre, P.F., Reinhart, K., Cuthbertson, B.H., Payen, D. and Briegel, J. (2008) Hydrocortisone therapy for patients with septic shock. N Engl J Med, 358, 111-124.
Talwar, S., Munson, P.J., Barb, J., Fiuza, C., Cintron, A.P., Logun, C., Tropea, M., Khan, S., Reda, D., Shelhamer, J.H., Danner, R.L. and Suffredini, A.F. (2006) Gene expression profiles of peripheral blood leukocytes after endotoxin challenge in humans. Physiol Genomics, 25, 203-215.
van den Berghe, G., Wouters, P., Weekers, F., Verwaest, C., Bruyninckx, F., Schetz, M., Vlasselaers, D., Ferdinande, P., Lauwers, P. and Bouillon, R. (2001) Intensive insulin therapy in the critically ill patients. N Engl J Med, 345, 1359-1367.
Varpula, M., Pulkki, K., Karlsson, S., Ruokonen, E. and Pettila, V. (2007) Predictive value of N-terminal pro-brain natriuretic peptide in severe sepsis and septic shock. Crit Care Med, 35, 1277-1283.
Vincent, J.L., de Mendonca, A., Cantraine, F., Moreno, R., Takala, J., Suter, P.M., Sprung, C.L., Colardyn, F. and Blecher, S. (1998) Use of the SOFA score to assess the incidence of organ dysfunction/failure in intensive care units: results of a multicenter, prospective study. Working group on "sepsis-related problems" of the European Society of Intensive Care Medicine. Crit Care Med, 26,1793-1800**.**
Vogl, T., Propper, C., Hartmann, M., Strey, A., Strupat, K., van den Bos, C., Sorg, C. and Roth, J. (1999) S100A12 is expressed exclusively by granulocytes and acts independently from MRP8 and MRP14. J Biol Chem, 274, 25291-25296.
Vogl, T., Tenbrock, K., Ludwig, S., Leukert, N., Ehrhardt, C., van Zoelen, M.A., Nacken, W., Foell, D., van der Poll, T., Sorg, C. and Roth, J. (2007) Mrp8 and Mrp14 are endogenous activators of Toll-like receptor 4, promoting lethal, endotoxin-induced shock. Nat Med, 13, 1042-1049.

## Claims

1. A method for *in vitro* establishing a prognosis for a subject in severe sepsis with at least two organ failures or in septic shock with at least two organ failures, comprising the following steps:
(i) from a plasma sample from said subject, measuring the level of the S100A8/A9 complex,
(ii) comparing said level to a predetermined threshold,
wherein a level of the S100A8/A9 complex above said predetermined threshold is indicative of a bad prognosis and a level of the S100A8/A9 complex below said predetermined threshold is indicative of a good prognosis.

2. The method of claim 1, wherein said biological sample has been collected at day 0, day 1 or day 2 after the onset of the second organ failure.

3. The method of claim 1 or claim 2, wherein said predetermined threshold is in the range 7-9 µg/ml.

4. The method of any of claims 1 to 3, wherein said predetermined threshold is in the range from 7.8 to 8.3 µg/ml.

5. The method of any of claims 1 to 4, wherein said predetermined threshold is 8.1 µg/ml.

6. The method of any of claims 1 to 5, wherein said measure in step (i) is done by an immunoassay.

7. The method of claim 6, wherein said immunoassay is performed with an antibody which specifically binds to the S100A8/A9 complex.

8. The method of claim 6 or claim 7, wherein said immunoassay is an ELISA assay.

9. The method of any of claims 1 to 8, further comprising a step of measuring the level of at least one other species in said plasma sample, wherein said other species is selected from the group consisting of procalcitonin (PCT), N-terminal pro-brain natriuretic peptide (BNP), soluble triggering receptor expressed on myeloid cells-1 (sTREM), IL-6 and sRAGE, and a step of comparing said level to a predetermined threshold.

10. A method for testing the efficiency of a pharmaceutical treatment for improving outcome of severe syndromes with at least two organ failures, comprising the following steps:
(i) selecting a patient in severe sepsis with at least two organ failures or in septic shock with at least two organ failures, and determining the level of S100A8/A9 complex in a plasma sample from said patient obtained before the beginning of said pharmaceutical treatment;
(ii) from at least another sample from said patient, obtained after the beginning of said pharmaceutical treatment, determining the level of S100A8/A9 complex;
(iii) comparing said obtained values;
wherein a decrease of S100A8/A9 complex level following the beginning of the pharmaceutical treatment indicates that said treatment has been beneficial to the patient and is likely to improve outcome of severe syndromes with at least two organ failures.

11. Use of a kit for performing the method according to any of claims 1 to 9, comprising an antibody specific for the S100A8/A9 complex and a notice of use explaining the predictive value of the plasma level of S100A8/A9 complex on the outcome of septic shock.

## Patentansprüche

1. Verfahren zur *In-vitro*-Erstellung einer Prognose für ein Subjekt bei schwerer Sepsis mit Versagen von mindestens zwei Organen oder bei septischem Schock mit Versagen von mindestens zwei Organen, umfassend die folgenden Schritte:
(i) Messen des Spiegels des S100A8/A9-Komplexes aus einer Plasmaprobe vom Subjekt,
(ii) Vergleichen des Spiegels mit einer vorbestimmten Schwelle,
wobei ein Spiegel des S100A8/A9-Komplexes über der vorbestimmten Schwelle eine schlechte Prognose anzeigt, und ein Spiegel des S100A8/A9-Komplexes unter der vorbestimmten Schwelle eine gute Prognose anzeigt.

2. Verfahren nach Anspruch 1, wobei die biologische Probe an Tag 0, Tag 1 oder Tag 2 nach Beginn des Versagens des zweiten Organs entnommen wurde.

3. Verfahren nach Anspruch 1 oder Anspruch 2, wobei die vorbestimmte Schwelle im Bereich von 7 bis 9 µg/ml liegt.

4. Verfahren nach einem der Ansprüche 1 bis 3, wobei die vorbestimmte Schwelle im Bereich von 7,8 bis 8,3 µg/ml liegt.

5. Verfahren nach einem der Ansprüche 1 bis 4, wobei die vorbestimmte Schwelle 8,1 µg/ml ist.

6. Verfahren nach einem der Ansprüche 1 bis 5, wobei die Messung in Schritt (i) durch einen Immunassay erfolgt.

7. Verfahren nach Anspruch 6, wobei der Immunassay mit einem Antikörper durchgeführt wird, der spezifisch an den S100A8/A9-Komplex bindet.

8. Verfahren nach Anspruch 6 oder Anspruch 7, wobei es sich bei dem Immunassay um einen ELISA-Assay handelt.

9. Verfahren nach einem der Ansprüche 1 bis 8, ferner umfassend einen Schritt des Messens des Spiegels mindestens einer anderen Spezies in der Plasmaprobe, wobei die andere Spezies aus der Gruppe bestehend aus Procalcitonin (PCT), N-terminalem pro-Brain natriuretischem Peptid (BNP), löslichem Auslösungsrezeptor exprimiert auf Knochenmarkzellen-1 (sTREM), IL-6 und sRAGE ausgewählt ist, und einen Schritt des Vergleichens des Spiegels mit einer vorbestimmten Schwelle.

10. Verfahren zum Prüfen der Wirksamkeit einer pharmazeutischen Behandlung zur Verbesserung des Ergebnisses von schweren Syndromen mit Versagen von mindestens zwei Organen, umfassend die folgenden Schritte:
(i) Auswählen eines Patienten bei schwerer Sepsis mit Versagen von mindestens zwei Organen oder bei septischem Schock mit Versagen von mindestens zwei Organen und Bestimmen des Spiegels des S100A8/A9-Komplexes in einer vor Beginn der pharmazeutischen Behandlung vom Patienten erhaltenen Plasmaprobe;
(ii) Bestimmen des Spiegels des S100A8/A9-Komplexes aus mindestens einer anderen vom Patienten nach Beginn der pharmazeutischen Behandlung erhaltenen Probe;
(iii) Vergleichen der erhaltenen Werte;
wobei eine Senkung des Spiegels des S100A8/A9-Komplexes nach Beginn der pharmazeutischen Behandlung darauf hinweist, dass die Behandlung für den Patienten günstig war und das Ergebnis von schweren Syndromen mit Versagen von mindestens zwei Organen wahrscheinlich verbessert.

11. Verwendung eines Kits zum Durchführen des Verfahrens nach einem der Ansprüche 1 bis 9, umfassend einen Antikörper, der für den S100A8/A9-Komplex spezifisch ist, und einen Gebrauchshinweis, der den Vorhersagewert des Plasmaspiegels des S100A8/A9-Komplexes über das Ergebnis eines septischen Schocks erläutert.

## Revendications

1. Procédé pour établir *in vitro* un pronostic pour un individu en sepsie sévère avec au moins deux défaillances d'organe ou en choc septique avec au moins deux défaillances d'organe, comprenant les étapes suivantes :
(i) à partir d'un échantillon de plasma provenant dudit individu, la mesure du niveau du complexe S100A8/A9,
(ii) la comparaison dudit niveau à un seuil prédéterminé,
dans lequel un niveau du complexe S100A8/A9 au-dessus dudit seuil prédéterminé est indicatif d'un mauvais pronostic et un niveau du complexe S100A8/A9 sous ledit seuil prédéterminé est indicatif d'un bon pronostic.

2. Procédé selon la revendication 1, dans lequel ledit échantillon biologique a été collecté au jour 0, au jour 1 ou au jour 2 après le début de la seconde défaillance d'organe.

3. Procédé selon la revendication 1 ou la revendication 2, dans lequel ledit seuil prédéterminé se trouve dans la plage de 7 à 9 µg/ml.

4. Procédé selon l'une quelconque des revendications 1 à 3, dans lequel ledit seuil prédéterminé se trouve dans la plage de 7,8 à 8,3 µg/ml.

5. Procédé selon l'une quelconque des revendications 1 à 4, dans lequel ledit seuil prédéterminé est de 8,1 µg/ml.

6. Procédé selon l'une quelconque des revendications 1 à 5, dans lequel ladite mesure à l'étape (i) est effectuée par un immunoessai.

7. Procédé selon la revendication 6, dans lequel ledit immunoessai est effectué avec un anticorps qui est spécifiquement lié au complexe S100A8/A9.

8. Procédé selon la revendication 6 ou la revendication 7, dans lequel ledit immunoessai est un essai ELISA.

9. Procédé selon l'une quelconque des revendications 1 à 8, comprenant en outre une étape de mesure du niveau d'au moins une autre espèce dans ledit échantillon de plasma, dans lequel ladite autre espèce est choisie dans le groupe constitué de procalcitonine (PCT), de peptide natriurétique de type B (BNP) N-terminal, de récepteur de déclenchement soluble exprimé sur des cellules myéloïdes-1 (sTREM), d'IL-6 et de sRAGE, et une étape de comparaison dudit niveau à un seuil prédéterminé.

10. Procédé pour tester l'efficacité d'un traitement pharmaceutique pour améliorer l'évolution de syndromes sévères avec au moins deux défaillances d'organe, comprenant les étapes suivantes :
(i) la sélection d'un patient en sepsie sévère avec au moins deux défaillances d'organe ou en choc septique avec au moins deux défaillances d'organe, et la détermination du niveau de complexe S100A8/A9 dans un échantillon de plasma provenant dudit patient obtenu avant le début dudit traitement pharmaceutique ;
(ii) à partir d'au moins un autre échantillon provenant dudit patient, obtenu après le début dudit traitement pharmaceutique, la détermination du niveau de complexe S100A8/A9 ;
(iii) la comparaison desdites valeurs obtenues ;
dans lequel une baisse du niveau de complexe S100A8/A9 suivant le début du traitement pharmaceutique indique que ledit traitement a été bénéfique pour le patient et a des chances d'améliorer l'évolution de syndromes sévères avec au moins deux défaillances d'organe.

11. Utilisation d'une trousse pour réaliser le procédé selon l'une quelconque des revendications 1 à 9, comprenant un anticorps spécifique pour le complexe S100A8/A9 et une notice d'utilisation expliquant la valeur prédictive du niveau de plasma de complexe S100A8/A9 sur l'évolution de choc septique.
